# FASCICULE DE BREVET EUROPEEN

(11) **EP 4 416 161 B1**
(45) Date de publication et mention de la délivrance du brevet: **11.03.2026**
(21) Numéro de dépôt: 22802578.9
(22) Date de dépôt: 12.10.2022
(51) Int. Cl.: C07H 1/00, C07H 15/04, C07H 15/10

(54) **PROCÉDÉ DE PRÉPARATION D'ALKYLPOLYGLYCOSIDES FAIBLEMENT COLORÉS AVEC PRÉ-NEUTRALISATION DU MILIEU RÉACTIONNEL**
VERFAHREN ZUR HERSTELLUNG VON NIEDRIG GEFÄRBTEN ALKYLPOLYGLYCOSIDEN MIT VORNEUTRALISIERUNG DES REAKTIONSMEDIUMS
PROCESS FOR THE PREPARATION OF LOW COLORED ALKYLPOLYGLYCOSIDES WITH PRE-NEUTRALIZATION OF THE REACTION MEDIUM

(30) Priorité: 13.10.2021 FR 2110843
(43) Date de publication de la demande: 21.08.2024
(73) Titulaire: Société d'Exploitation de Produits pour les Industries Chimiques SEPPIC, 75321 Paris cedex 07 (FR)
(72) Inventeur: ILLOUS, Estelle, 81100 Castres (FR); DESSILLA, Stéphane, 81100 Castres (FR)
(74) Mandataire: Air Liquide
(86) Numéro de dépôt international: PCT/EP2022/078400
(87) Numéro de publication internationale: WO 2023/062077

(56) Documents cités:
- EP-A1- 0 077 167
- EP-A1- 0 092 876
- EP-A1- 0 338 151
- WO-A1-98/35975
- US-A- 5 576 425
- US-A- 5 681 938

## Description

La présente invention est relative à un procédé de préparation d'Alkyl Polyglycosides peu colorés (couleur inférieur à 1,5 vcs) impliquant des agents de neutralisation de type carbonate.

Les AlkylPolyGlycosides ou APG sont probablement les meilleurs exemples de tensioactifs biosourcés disponibles aujourd'hui sur le marché. Leurs structures moléculaires se caractérisent par la présence simultanée d'une tête hydrophile dérivée de sucres réducteurs (D-glucose, D-xylose ou D-Rhamnose sont les sucres réducteurs principalement disponibles à l'échelle industrielle) et d'une chaîne hydrocarbonée lipophile plus ou moins longue (cf. formule I : structure simplifiée d'un APG).

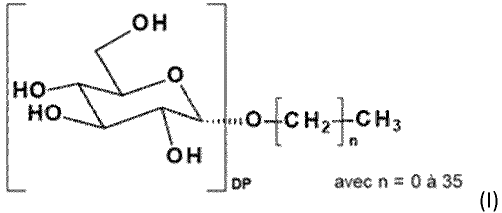

Leur procédé de fabrication à l'échelle industrielle est relativement simple et utilise comme matières premières i) le glucose ou le xylose ou le rhamnose cristallisé issus respectivement de l'hydrolyse totale d'amidon de blé, de maïs ou de pomme de terre ou de l'hydrolyse d'hémicelluloses de bois et ii) des alcools gras provenant de la filière oléochimique (hydrogénation d'esters méthyliques issus de la transestérification de triglycérides végétaux). Les réactions de glycosylation de Fischer consistent alors à relier ces deux matières premières entre elles en créant une liaison chimique covalente, comme par exemple dans la réaction (II) entre le glucose et un alcool.

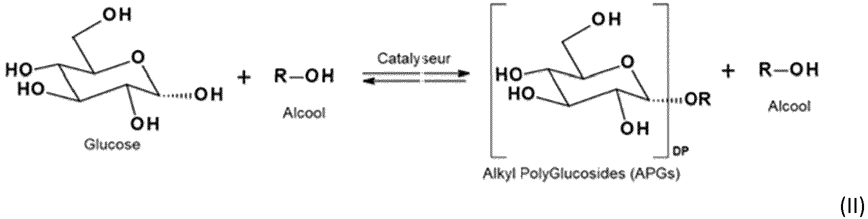

Pour réaliser cette réaction de glycolysation, un catalyseur acide d'origine minérale ou organique est nécessaire et un excès d'alcools est systématiquement introduit jouant ainsi le rôle de réactif et de solvant. En fin de réaction, les APG se retrouvent dispersés ou solubilisés dans l'excès d'alcool qui n'a pas réagi. Les APG se distinguent par la nature et la longueur de la chaîne alkyle hydrocarbonée R, ainsi que par leur Degré de Polymérisation moyen DP, supérieur à 1 mais inférieur ou égal à 2,5.

A l'issue de la phase réactionnelle de glycolysation, une étape de neutralisation est opérée afin de désactiver le catalyseur et de stopper la réaction.

Selon la longueur de la chaîne alkyle de l'alcool et l'utilisation associée, ledit alcool est soit éliminé soit conservé.

L'étape de neutralisation diffère selon la longueur de la chaîne alkyle hydrocarbonée. Dans le cas où celle-ci présente un nombre d'atomes de carbone inférieur à 12, la neutralisation est réalisée par une solution aqueuse d'hydroxyde de sodium. L'excès d'alcools gras présent en fin de glycosylation est ensuite éliminé par distillation sous vide poussé ou distillation moléculaire, ou par évaporation, généralement, à l'aide d'un évaporateur couche mince à film tombant, ou d'un évaporateur couche mince à court trajet, et le concentré d'APG recueilli est finalement solubilisé dans l'eau. Les produits commerciaux ainsi obtenus se présentent donc sous la forme de solutions aqueuses d'APG avec une concentration massique comprise entre 40 et 80%.

Dans le cas où la chaîne alkyle hydrocarbonée R présente un nombre d'atome de carbone supérieur ou égal à 12 , la neutralisation est généralement réalisée par l'hydroxyde de sodium ou par l'hydroxyde de potassium, seuls ou en combinaison avec un agent réducteur tel que décrit dans le brevet Européen publié sous le numéro EP0077167, dans la demande de brevet Européen publiée sous le numéro EP0338151A1, dans le brevet Européen EP0388857B1, comme par exemple le borohydrure de sodium (NaBH₄) ou l'hypophosphite de sodium (NaH₂PO₂). Le mélange d'APG et de l'excès d'alcools gras est isolé après neutralisation et est commercialisé tel quel. La proportion des APG et des Alcools gras dépend de la stœchiométrie molaire retenue au départ pour les matières premières et de leur réactivité. Toutefois, des proportions de 5 à 30% massique d'APG et de 70 à 95% d'alcools gras sont généralement observées. Les compositions obtenues peuvent se présenter sous forme d'un solide, comme par exemple sous la forme d'écailles ou de perles, ou sous une forme liquide, en fonction de la nature de la chaîne alkyle hydrocarbonée R.

Cependant, l'étape de neutralisation des APG dont la chaîne alkyle hydrocarbonée R comporte un nombre d'atomes de carbone supérieur ou égale à 12 par une base de l'état de la technique (exemple NaOH, KOH), pour atteindre une valeur du pH d'une dispersion à 5% massique dans l'eau du milieu neutralisé comprise entre 5,5 et 7,5, engendre une coloration importante du produit.

Par "mesure du pH d'une dispersion à 5% massique dans l'eau", on désigne au sens de la présente invention la méthode analytique de mesure de pH d'une dispersion d'une composition à base d'APG selon les dispositions de la norme NF EN 1262, ladite mesure est réalisée par mesure potentiométrique à l'aide d'une électrode pH (milieux aqueux) combinée et d'un pH mètre.

Cette coloration peut altérer les qualités organoleptiques des produits finis dans lesquels les compositions les APG sont introduites. C'est pour cela que des solutions sont apportées pour minimiser la coloration des compositions contenant les APG dont la chaîne alkyle hydrocarbonée R comporte un nombre d'atomes de carbone supérieur ou égale à 12. Deux leviers connus de l'état de la technique sont classiquement employés pour obtenir de telles compositions à base d'APG dont la chaîne alkyle hydrocarbonée R comporte un nombre d'atomes de carbone supérieur ou égale à 12 peu colorées (< 1,5 vcs).

Par "composition peu colorée", on désigne au sens de la présente invention une composition dont l'indice de couleur Gardner, tel que défini par la norme DIN-ISO 4630, est inférieur ou égal à 1,5 VCS. L'indice de couleur Gardner est mesuré en utilisant un colorimètre LICO 200/Dr LANGE (ou équivalent) qui effectue des mesures par transmission de la lumière sur tout milieu. Un tel colorimètre fonctionne avec une lampe halogène correspondant à l'illuminant normalisé C, défini par la norme DIN 5033 et avec un observateur normalisé doté d'un champ de vision de 2°. Pendant la mesure, un faisceau de rayonnement de référence compense les variations des valeurs enregistrées dues aux différences de lampe et de température.

Le premier levier consiste à associer un agent réducteur à la base utilisée. Parmi ces agents réducteurs, nous pouvons citer le borohydrure de sodium (NaBH₄) ou de l'hypophosphite de sodium (NaH₂PO₂). Cette solution n'est pas entièrement satisfaisante. En effet, bien que très efficace pour minimiser la coloration de la composition traitée, le NaBH₄ est un agent réducteur dangereux à manipuler et à mettre en œuvre (produit corrosif, dégagement d'hydrogène). Le NaH₂PO₂ est quant à lui très peu efficace même s'il est introduit à forte concentration.

Le second levier couramment utilisé et décrit dans l'état de la technique, pour minimiser la couleur des compositions à base d'APG, dont la chaîne alkyle hydrocarbonée R comporte un nombre d'atomes de carbone supérieur ou égal à 12, est la réalisation d'une décoloration au péroxyde d'hydrogène (H₂O₂) lors de l'étape de finition. Bien qu'efficace, cette étape est néanmoins fastidieuse car elle nécessite d'ajuster la valeur du pH d'une dispersion à 5% massique dans l'eau entre 7,0 et 7,5 tout en maintenant le pouvoir oxydant du milieu par ajouts de d'H₂O₂. Cette étape délicate à réaliser peut durer plusieurs heures et donc accroître significativement la durée de production, en diminuant la productivité.

D'autres documents de l'art antérieur divulguent d'autres procédés de préparation. Ainsi, EP0092876A1 décrit une préparation par transglycosylation comprenant une étape de neutralisation réalisée avec Na2CO3. US5681938A décrit un procédé de préparation d'APG comprenant une étape de réalisation réalisée avec une amine tertiaire. US5576425A divulgue une étape de neutralisation incluant des carbonates avec MgO comme base préférée. Enfin, WO98/35975A1 décrit une préparation d'APG comprenant l'utilisation d'un catalyseur sulfate binaire lui-même étant un mélange de H2SO4 et d'une base inorganique incluant les carbonates.

Le problème technique à résoudre est donc de trouver une alternative à la neutralisation des compositions d'APG dont la chaîne alkyle hydrocarbonée R comporte un nombre d'atomes de carbone supérieur ou égal à 12. Cette alternative doit être efficace et facile à mettre en œuvre, tout en garantissant une couleur inférieure ou égale à 1,5 vcs sans mettre en œuvre une étape de décoloration.

Une solution de la présente invention est un procédé de préparation d'une composition (C) de couleur inférieure ou égale à 1,5 vcs comprenant pour 100% de sa masse:
i) une quantité supérieure ou égale à 40% massique et inférieure ou égale à 95% massique, de préférence supérieure ou égale à 50% massique et inférieure ou égale à 95% massique, encore plus préférentiellement supérieure ou égale à 70% massique et inférieure ou égale à 90% massique d'un alcool de formule (I) :

   R-OH (I),

   dans laquelle R représente un radical hydrocarboné, linéaire ou ramifié, saturé ou insaturé, pouvant comporter au moins un fonction hydroxy, et comportant de douze à vingt-deux atomes de carbone, ou d'un mélange d'alcools de formule (I) ;
ii) une quantité supérieure ou égale à 5% massique et inférieure ou égale à 60% massique, de préférence supérieure ou égale à 5% massique et inférieure ou égale à 50% massique, et encore plus préférentiellement supérieure ou égale à 10% et inférieure ou égale à 30% massique d'une composition (C1) représentée par la formule (II) :

   R-O-(G)x-H (II),

   dans laquelle le reste G représente le reste d'un sucre réducteur, R représente un radical tel que défini dans la formule (I) et x, qui indique le degré moyen de polymérisation du reste G représente un nombre décimal supérieur à 1,05 et inférieur ou égal à 2,5, ou d'un mélange de compositions (C1) de formule (II);
étant entendu que la somme des proportions massiques des composés de formules (I) et (II) dans la composition (C) est égale à 100% massique,
ledit procédé comprenant successivement:
a) Une étape a) de glycosylation, consistant en une réaction entre au moins un alcool de formule (I) et au moins un sucre réducteur de formule (III) : H-O-(G)-H (III), en présence d'au moins un catalyseur acide (CA), à une température supérieure ou égale à 100°C et inférieure ou égale à 120°C, préférentiellement supérieure ou égale à 100°C et inférieure ou égale à 115°C, encore plus préférentiellement supérieure ou égale à 100°C et inférieure ou égale à 110°C, le catalyseur acide (CA) étant choisi parmi les éléments du groupe constitué par l'acide sulfurique, l'acide chlorhydrique, l'acide phosphorique, l'acide nitrique, l'acide hypophosphoreux, l'acide méthane-sulfonique, l'acide para- toluène sulfonique, l'acide trifluorométhane sulfonique et les résines échangeuses d'ions acides,
b) Une étape b) de pré-neutralisation du milieu réactionnel issu de l'étape a), la pré-neutralisation étant réalisée de manière à obtenir un milieu réactionnel dont une dispersion à 5% massique dudit milieu réactionnel dans l'eau présente une valeur de pH comprise entre 3,5 et 5,5,
c) Une étape c) d'élimination du sucre réducteur de formule (III), qui n'a pas réagi à l'étape a), du milieu réactionnel pré-neutralisé obtenu à l'étape b),
d) Une étape d) de neutralisation du milieu réactionnel issu de l'étape c) avec une solution aqueuse comprenant un agent basique (Ab) choisi parmi les éléments du groupe constitué par :
   ▪ les carbonates de formule (IVa) :

      XnCO₃ (IVa),

      dans laquelle X représente un atome de sodium ou de potassium et n est un nombre entier égal à 2, ou bien X représente un atome de calcium ou un atome de magnésium et n est un nombre entier égal à 1, ou
   ▪ les hydrogénocarbonates de formule (IVb) :

      Y(HCO₃)m (IVb),

      dans laquelle Y représente un atome de sodium ou de potassium et m est un nombre entier égal à 1, ou bien Y représente un atome de calcium ou un atome de magnésium et m est un nombre entier égal à 2,
      la naturalisation étant réalisée de manière à obtenir un milieu réactionnel dont une dispersion à 5% massique dudit milieu réactionnel dans l'eau présente une valeur de pH comprise entre 5,5 et 7,5, et
e) Une étape e) de récupération d'au moins une composition (C) de couleur inférieure ou égale à 1,5 vcs.

L'indice de couleur caractérisant la composition (C) préparée selon le procédé objet de la présente invention, est l'indice de couleur Gardner, tel que défini par la norme DIN-ISO 463. L'indice de couleur Gardner est mesuré en utilisant un colorimètre LICO 200/Dr LANGE (ou équivalent) qui effectue des mesures par transmission de la lumière sur tout milieu. Un tel colorimètre fonctionne avec une lampe halogène correspondant à l'illuminant normalisé C, défini par la norme DIN 5033 et avec un observateur normalisé doté d'un champ de vision de 2°. Pendant la mesure, un faisceau de rayonnement de référence compense les variations des valeurs enregistrées dues aux différences de lampe et de température.

L'unité d'expression de l'indice de couleur Gardner, caractérisant la composition (C) préparée selon le procédé objet de la présente invention, est le VCS.

Selon le cas, le procédé selon l'invention peut présenter une ou plusieurs des caractéristiques suivantes :
- ladite composition (C1) consiste en un mélange de composés représentés par les formules (II1), (II2), (II3), (II4) et (II5):

   R-O-(G)1-H (II1),

   R-O-(G)2-H (II2),

   R-O-(G)3-H (II3),

   R-O-(G)4-H (II4),

   R-O-(G)5-H (II5),

   dans les proportions molaires respectives a1, a2, a3, a4 et a5, telles que:
   ▪ la somme: a1+ a2 + a3 + a4 + a5 est égale à 1, et
   ▪ la somme a1 + 2a2 + 3a3 + 4a4 + 5a5 est égale à x ;
- la composition (C) comprend une quantité inférieure ou égale à 2% massique, plus particulièrement inférieure ou égale à 1% massique du sucre réducteur de formule (III) :

   H-O-(G)-H (III) ;

   étant entendu que la somme des proportions massiques des composés de formules (I), (II) et (III) dans la composition (C) est égale à 100% massique ;
- l'agent basique (Ab) compris dans la solution aqueuse utilisée dans l'étape d) de neutralisation est choisi parmi le carbonate de sodium (Na₂CO₃) ou l'hydrogénocarbonate de sodium (NaHCO₃) ;
- l'étape b) de pré-neutralisation est réalisée avec au moins un des composés suivants : hydroxyde de sodium (NaOH), hydroxyde de potassium (KOH), hydroxyde d'ammonium (NH₄OH), monoéthanolamine, diéthanolamine, triéthanolamine et triéthylamine ;
- l'étape b) de pré-neutralisation est réalisée avec un des composés suivants : carbonate de sodium (Na₂CO₃), hydrogénocarbonate de sodium (NaHCO₃), et carbonate de calcium (CaCO₃₎;
- le sucre réducteur de formule (III) choisi pour la glycolysation de l'étape a) est choisi parmi les éléments du groupe constitué par le glucose, le xylose, l'arabinose, le rhamnose ;
- l'étape c) d'élimination du sucre réducteur de formule (III) est réalisée par filtration, centrifugation ou décantation ;
- à l'étape d) la solution aqueuse d'agent basique (Ab) comprend entre 10 % et 25% massique dudit agent basique (Ab) ;
- l'étape a) comprend les sous-étapes successives suivantes :
   i) Introduction d'un alcool de formule (I) ou d'un mélange d'alcools de formule (I), dans un réacteur (Ré) équipé d'une agitation mécanique et d'un dispositif de mise sous vide ;
   ii) Chauffage de l'alcool de formule (I) à une température comprise entre 80°C et 90°C sous agitation mécanique;
   iii) Chargement du sucre réducteur de formule (III) dans le réacteur (Ré) ;
   iv) Introduction du catalyseur acide (CA) dans le réacteur (Ré),
   v) Chauffage sous vide partiel du milieu réactionnel issu de la sous-étape iv) et présent dans le réacteur (Ré) à une température comprise entre 100°Cet 110°C pendant la durée de la réaction, et
   vi) Refroidissement du milieu réactionnel issu de la sous-étape v) à une température comprise entre70°C et 80°C ;
- le radical R est choisi parmi les radicaux suivants : lauryle (ou n-dodécyle), myristyle (ou n-tétradécyle ), n-pentadécyle, cétyle (ou n-hexadécyle), n-heptadécyle, stéaryle (ou n-octadécyle), palmitoléyle (ou 9-hexadécényle), oléyle (ou 9-octadécényle), linoléyle (9,12-octadecadiényle), linolényle (ou 6,9,12-octadécatriényle) nonadécyle, arachidyle (ou n-eicosyle), béhényle (ou n-docosyle), érucyle (13-docosényle), ou 12-hydroxystéaryle ;
- le radical R est choisi parmi les radicaux suivants : 2-hexyl octyle, 2-hexyl décyle, 2-hexyl dodécyle, 2-octyl décyle, 2-octyl dodécyle, 2-décyl tétradécyle, isostéaryle (ou 16-méthyl heptadécyle) ou isomyristyle (ou 13-méthyl tridécyle) ;
- le catalyseur acide (CA) est choisi parmi les éléments du groupe constitué par l'acide sulfurique, l'acide chlorhydrique, l'acide phosphorique, l'acide nitrique, l'acide hypophosphoreux, l'acide méthane-sulfonique, l'acide para-toluène sulfonique, l'acide trifluorométhane sulfonique et les résines échangeuses d'ions acides ;
- pendant les sous-étapes ii) à iv) le réacteur (Ré) est inerté sous azote ;
- le procédé comprend entre les sous-étapes ii) et iii) une étape de mise sous vide, de préférence à une pression inférieure à ou égale à 50 millibars ;
- la sous-étape vi) est réalisée à pression atmosphérique.

L'emploi de carbonates de formule (IVa) ou d'hydrogénocarbonates de formule (IVb) ne contribue pas à augmenter la couleur de la composition (C) (la présence d'un agent réducteur n'étant pas alors nécessaire) tout en neutralisant au pH désiré d'une dispersion à 5% massique de la composition (C) (dont la valeur est comprise entre 5,5 et 7,5). L'utilisation d'un agent basique (Ab) permet d'éviter une étape de décoloration impliquant l'utilisation d'un agent péroxyde, ou autres, puisqu'elle permet d'atteindre une couleur inférieure ou égale à 1,5 vcs.

Par sucre réducteur, on désigne dans la définition de la formule (II) et dans la définition de la formule (III), les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990.

La structure oligomérique (G)x présente dans la formule (II), peut se présenter sous toutes formes d'isoméries, qu'il s'agisse d'isomérie optique, d'isomérie géométrique ou d'isomérie de position ; elle peut aussi représenter un mélange d'isomères.

Dans la formule (II) telle que définie ci-dessus, le radical R est lié à G par le carbone anomérique du reste saccharide, de manière à former une fonction acétal.

Selon un aspect particulier de la présente invention, dans la définition des composés de formules (II) et de formule (III), G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le rhamnose, dextrane ou le tallose.

Selon un aspect particulier de la présente invention, dans la définition des composés de formule (II), G représente le reste d'un sucre réducteur choisi parmi les restes du glucose, du xylose, de l'arabinose ou du rhamnose, et x représente un nombre décimal supérieur ou égal à 1,05 et inférieur ou égal à 2,5.

Selon un aspect encore plus particulier de la présente invention, dans la définition des composés de formule (II), G représente le reste d'un sucre réducteur est choisi parmi les restes du glucose, du xylose, de l'arabinose ou du rhamnose, et x représente un nombre décimal supérieur ou égal à 1,05 et inférieur ou égal à 2,0, et encore plus particulièrement supérieur ou égal à 1,25 et inférieur ou égal à 2,0.

Selon un autre aspect particulier de la présente invention, le sucre réducteur de formule (III) est choisi parmi les éléments du groupe constitué par le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le rhamnose, dextrane ou le tallose.

Selon un aspect plus particulier de la présente invention, le sucre réducteur de formule (III) est choisi parmi le glucose, le xylose, l'arabinose ou le rhamnose.

Le procédé selon l'invention consiste à réaliser une pré-neutralisation du milieu en fin de réaction de glycosylation pour atteindre une valeur du pH d'une dispersion à 5% massique dudit milieu dans l'eau comprise entre 3,5 et 5,5. Cette pré-neutralisation peut être réalisée par un carbonate de métal alcalin ou de métal alcalino-terreux, un hydrogénocarbonate de métal alcalin ou de métal alcalino-terreux ou toutes autres bases connues par l'homme du métier. Le sucre réducteur de formule (III) résiduel est ensuite éliminé par filtration et un ajout d'une solution aqueuse d'un agent basique (Ab) est réalisé pour atteindre une valeur du pH de la dispersion à 5% massique dans l'eau de la composition (C) compris entre 5,5 et 7,5.

Selon un aspect particulier, le procédé a pour objet la préparation d'une composition (C) de couleur inférieure ou égale à 1,5 VCS, comprenant pour 100% de sa masse :
- de 45% à 55% massique d'un mélange (M1) d'alcools de formule (I) comprenant pour 100% de la masse dudit mélange (M1), 50% massique d'un alcool de formule (I) dans laquelle R représente le radical n-hexadécyle et 50% massique d'un alcool de formule (I) dans laquelle R représente le radical n-octadécyle
- de 45% à 54% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle
- moins de 1% massique de glucose.

Selon un aspect particulier, le procédé a pour objet la préparation d'une composition (C) de couleur inférieure ou égale à 1,5 VCS, comprenant pour 100% de sa masse :
- de 45% à 55% massique d'un mélange (M'1) d'alcools de formule (I) comprenant pour 100% de la masse dudit mélange (M'1), 70% massique d'un alcool de formule (I) dans laquelle R représente le radical n-hexadécyle et 30% massique d'un alcool de formule (I) dans laquelle R représente le radical n-octadécyle
- de 45% à 54% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange (M"1) d'alcools de formule (I) comprenant pour 100% de la masse dudit mélange (M"1), 50% massique d'un alcool de formule (I) dans laquelle R représente le radical n-hexadécyle et 50% massique d'un alcool de formule (I) dans laquelle R représente le radical n-octadécyle
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange (M'"1) d'alcools de formule (I) comprenant pour 100% de la masse dudit mélange (M‴1), 70% massique d'un alcool de formule (I) dans laquelle R représente le radical n-hexadécyle et 30% massique d'un alcool de formule (I) dans laquelle R représente le radical n-octadécyle
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-hexadécyle et le radical n-octadécyle
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un alcool de formule (I) dans laquelle R représente le radical n-tétradécyle ;
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-tétradécyle ;
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange d'alcools de formule (I) dans laquelle R représente le radical n-dodécyle, le radical n-tétradécyle, le radical n-hexadécyle et le radical n-octadécyle ;
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical représente le radical n-dodécyle, le radical n-tétradécyle, le radical n-hexadécyle, et le radical n-octadécyle ;
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange d'alcools de formule (I) dans laquelle R représente le radical n-eicosyl et le radical n-docosyle ;
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-eicosyl et le radical n-docosyle ;
- moins de 1% massique de glucose.

Selon un aspect particulier, le procédé a pour objet la préparation d'une composition (C) de couleur inférieure ou égale à 1,5 VCS, comprenant pour 100% de sa masse :
- de 75% à 90% massique d'un mélange d'alcools de formule (I) dans laquelle R représente le radical n-dodécyle, le radical n-tétradécyle, le radical n-hexadécyle, le radical n-eicosyl et le radical n-docosyle ;
- de 10% à 24% massique d'au moins une composition (C1) représentée par la formule (II) dans laquelle G représente le radical glucosyl ou α,β-D-glucopyranosyl, obtenu à partir de la suppression du groupe hydroxyl hémiacétal du α,β-D-glucopyranose, x représente un nombre décimal supérieur ou égal à 1,05 et inférieur ou égal à 2,0, R représente le radical n-dodécyle, le radical n-tétradécyle, le radical n-hexadécyle, le radical n-eicosyl et le radical n-docosyle ;
- moins de 1% massique de glucose.

Selon un aspect particulier, le procédé a pour objet la préparation d'une composition (C) de couleur inférieure ou égale à 1,5 VCS, comprenant pour 100% de sa masse :
- de 70% à 90% massique d'un mélange d'alcools de formule (I) dans laquelle R représente le radical n-dodécyle, le radical n-tétradécyle, le radical n-hexadécyle, le radical n-eicosyl et le radical n-docosyle ;
- de 10% à 29% massique d'au moins une composition (C1) représentée par la formule (II) dans laquelle G représente le radical xylosyl ou α,β-D-xylopyranosyl, obtenu à partir de la suppression du groupe hydroxyl hémiacétal du α,β-D-xylopyranose, x représente un nombre décimal supérieur ou égal à 1,05 et inférieur ou égal à 2,0, R représente le radical 2-octyl dodécyle ;
- moins de 1% massique de xylose.

Selon un aspect particulier, l'agent basique (Ab) présent dans la solution aqueuse est le carbonate de potassium de formule (IVa) dans laquelle X représente l'atome de potassium et n est égal à 2.

Selon un aspect particulier, l'agent basique (Ab) présent dans la solution aqueuse est l'hydrogénocarbonate de sodium de formule (IVb) dans laquelle Y représente un atome de sodium et m est égal à 1.

Selon un aspect particulier, le catalyseur acide (CA) est choisi parmi les éléments du groupe constitué par l'acide sulfurique, l'acide phosphorique, l'acide hypophosphoreux, l'acide méthane-sulfonique, l'acide p-toluène sulfonique.

### EXEMPLES

Comparaison de l'effet de l'agent neutralisant sur la couleur d'une composition d'alcools gras et d'alkylpolyglucosides lorsque l'agent basique neutralisant utilisé est un carbonate selon l'invention ou la soude (agent neutralisant comparatif).

Des comparaisons entre un carbonate et la soude comme agent neutralisant ont été effectuées. Pour ceci, des réactions de glycosylation ont été menées à partir de glucose cristallisé et de différents alcools gras sous forme de coupes ou purs : coupe cétéarylique C-16/18, coupe arachydilique/béhénique C-20/22, tétradécanol-1 (alcool C-14) et dodécanol-1 (alcool C-12).

### 1. Exemples selon l'invention

### Exemple 1.1 (Coupe d'alcools 16/18 et Na₂CO₃ comme agent pré-neutralisant et agent neutralisant) selon l'invention

### ETAPE 1 : Réaction de Glycosylation :

967,4 g d'alcool cétéarylique (C-16/18) sont chargés dans un réacteur équipé d'une agitation mécanique et d'un montage de distillation sous vide. L'alcool est fondu à 85°C et mis sous agitation et sous barbotage d'azote. Le milieu est mis sous vide à des pressions inférieures à 50 Torrs. Une quantité de glucose anhydre sous forme de poudre est ajoutée de sorte à ce que le rapport molaire entre les alcools gras et le glucose soit de 6/1. Le milieu est inerté sous azote. Pour démarrer la réaction d'éthérification 0,9 g d'une solution aqueuse de H₃PO₂ à 50 % puis 1,1 g d'une solution aqueuse de H₂SO₄ à 98 % sont ajoutés et la température est augmentée et maintenue à 105°C. La réaction est poursuivie pendant une durée de 5h45.

### ETAPE 2 : Neutralisation du milieu réactionnel :

Le milieu est ensuite refroidi à 80°C à pression atmosphérique puis pré-neutralisé en introduisant 2,21 g d'une solution aqueuse de Na₂CO₃ à 25 %. Le produit est ensuite introduit dans un flacon en verre et placé à l'étuve à 80°C pendant 5 heures afin de décanter le glucose résiduel.Le produit (phase supérieure) est ensuite filtré sur papier filtre (environ 10 µm). Une dispersion à 5% massique dans l'eau montre une valeur de pH de 4,7 et le produit une couleur de 0,6 VCS. Le produit est ensuite neutralisé en introduisant 4,61 g d'une solution aqueuse de Na₂CO₃ à 25 %. Le produit est ensuite introduit dans un flacon en verre et placé à l'étuve à 80°C pendant 24 heures afin de décanter le glucose résiduel. Le produit (phase supérieure) est récupéré et référencé (Composition 1).

### Analyses :

- La valeur du pH d'une dispersion à 5% massique dans l'eau de la Composition 1 est de 6,8, et
- la mesure de la couleur de Composition 1 est de 0,5 VCS.

### Exemple 1.2 (Coupe d'alcools 16/18 et NaOH comme agent pré-neutralisant et Na₂CO₃ comme agent neutralisant) selon l'invention

### ETAPE 1 : Réaction de Glycosylation :

1364,8 g d'alcool cétéarylique (C-16/18) sont chargés dans un réacteur équipé d'une agitation mécanique et d'un montage de distillation sous vide. L'alcool est fondu à 85°C et mis sous agitation et sous barbotage d'azote. Le milieu est mis sous vide à des pressions inférieures à 50 Torrs. 179,2 g de glucose anhydre sous forme de poudre sont ajoutés. Le milieu est inerté sous azote. Pour démarrer la réaction d'éthérification 1,2 g d'une solution aqueuse de H₃PO₂ à 50 % puis 1,6 g d'une solution aqueuse de H₂SO₄ à 98 % sont ajoutés et la température est augmentée et maintenue à 105°C. La réaction est poursuivie pendant 5h45.

### ETAPE 2 : Neutralisation du milieu réactionnel :

Le milieu est refroidi à 80°C à pression atmosphérique puis pré-neutralisé en introduisant 3,4 g d'une solution aqueuse de NaOH à 25 % sous agitation. Le produit est ensuite introduit dans un flacon en verre et placé à l'étuve à 80°C pendant 5 heures afin de décanter le glucose résiduel. Le produit (phase supérieure) est ensuite filtré sur papier filtre (^{~} 10 µm). Une dispersion à 5% massique dans l'eau montre une valeur de pH de 4,5 et le produit une couleur de 1,0 VCS. Le produit (880 g) est ensuite neutralisé à 85°C en réacteur sous agitation, en introduisant 3,57 g d'une solution aqueuse de Na₂CO₃ à 10 %. Le produit est récupéré et référencé (Composition 2).

### Analyses :

- La valeur du pH d'une dispersion à 5% massique dans l'eau de la Composition 2 est de 7,2, et
- la mesure de la couleur de Composition 2 est de 0,9 VCS.

### Exemple 1.3 (Coupe d'alcools 16/18 et NaOH comme agent pré-neutralisant et NaHCO₃ comme agent neutralisant) selon l'invention

### ETAPE 1 : Réaction de Glycosylation :

271,1 g d'alcool cétéarylique (C-16/18) sont chargés dans un réacteur équipé d'une agitation mécanique et d'un montage de distillation sous vide. L'alcool est fondu à 85°C et mis sous agitation et sous barbotage d'azote. Le milieu est mis sous vide à des pressions inférieures à 50 Torrs. 35,7 g de glucose anhydre sous forme de poudre sont ajoutés. Le milieu est inerté sous azote. Pour démarrer la réaction d'éthérification 0,2 g d'une solution aqueuse de H₃PO₂ à 50 % puis 0,3 g d'une solution aqueuse de H₂SO₄ à 98 % sont ajoutés et la température est augmentée et maintenue à 105°C. La réaction est poursuivie pendant 5h45.

### ETAPE 2 : Neutralisation du milieu réactionnel :

Le milieu est refroidi à 85°C à pression atmosphérique puis pré-neutralisé en introduisant 0,4 g d'une solution aqueuse de NaOH à 25 % sous agitation. Le produit est ensuite introduit dans un flacon en verre et placé à l'étuve à 80°C pendant 24 heures afin de décanter le glucose résiduel. Une dispersion à 5% massique dans l'eau montre une valeur de pH de 3,5 et le produit une couleur de 0,5 VCS. Le produit (173,8 g) est ensuite neutralisé en réacteur à 85°C sous agitation en introduisant 1,9 g d'une solution aqueuse de NaHCO₃ à 8 %. Le produit est récupéré et référencé (Composition 3).

### Analyses :

- La valeur du pH d'une dispersion à 5% massique dans l'eau de la Composition 3 est de 6,3, et
- la mesure de la couleur de Composition 3 est de 0,4 VCS.

### Exemple 1.4 (Coupe d'alcools 20/22 et NaOH comme agent pré-neutralisant et Na₂CO₃ comme agent neutralisant) selon l'invention

### ETAPE 1 : Réaction de Glycosylation :

284,7 g d'alcools bénéhylique/arachidylique (C-20/22) sont chargés dans un réacteur équipé d'une agitation mécanique et d'un montage de distillation sous vide. L'alcool est fondu à 85°C et mis sous agitation et sous barbotage d'azote. Le milieu est mis sous vide à des pressions inférieures à 50 Torrs. 29,2 g de glucose anhydre sous forme de poudre sont ajoutés. Le milieu est inerté sous azote. Pour démarrer la réaction d'éthérification 0,3 g d'une solution aqueuse de H₃PO₂ à 50 % puis 0,4 g d'une solution aqueuse de H₂SO₄ à 98 % sont ajoutés et la température est augmentée et maintenue à 105°C. La réaction est poursuivie pendant 4h30.

### ETAPE 2 : Neutralisation du milieu réactionnel :

Le milieu est refroidi à 90°C à pression atmosphérique puis pré-neutralisé en introduisant 0,89 g d'une solution aqueuse de NaOH à 25 % sous agitation. Le produit est ensuite introduit dans un flacon en verre et placé à l'étuve à 80°C pendant 24 heures afin de décanter le glucose résiduel. Une dispersion à 5% massique dans l'eau montre une valeur de pH de 5,1 et le produit une couleur de 0,3 VCS.

175,4 g de produit (phase supérieure) est ensuite neutralisé à 90°C en réacteur sous agitation, en introduisant 0,61 g d'une solution aqueuse de Na₂CO₃ à 10 %.

Le produit est récupéré et référencé (Composition 4).

### Analyses :

- La valeur du pH d'une dispersion à 5% massique dans l'eau de la Composition 4 est de 6,8, et
- la mesure de la couleur de Composition 4 est de 0,5 VCS.

### Exemple 1.5 (Coupe d'alcools 20/22 et Na2CO3 comme agent pré-neutralisant et Na₂CO₃ comme agent neutralisant) selon l'invention

### ETAPE 1 : Réaction de Glycosylation :

391,9 g d'alcool bénéhylique/arachidylique (C-20/22) sont chargés dans un réacteur équipé d'une agitation mécanique et d'un montage de distillation sous vide. L'alcool est fondu à 85°C et mis sous agitation et sous barbotage d'azote. Le milieu est mis sous vide à des pressions inférieures à 50 Torrs. 40,1 g de glucose anhydre sous forme de poudre sont ajoutés. Le milieu est inerté sous azote. Pour démarrer la réaction d'éthérification 0,4 g d'une solution aqueuse de H₃PO₂ à 50 % puis 0,6 g d'une solution aqueuse de H₂SO₄ à 98 % sont ajoutés et la température est augmentée et maintenue à 105°C. La réaction est poursuivie pendant 4h30.

### ETAPE 2 : Neutralisation du milieu réactionnel :

Le milieu est refroidi à 90°C à pression atmosphérique puis pré-neutralisé en introduisant 2,1 g d'une solution aqueuse de Na₂CO₃ à 25 % sous agitation. Le produit est ensuite introduit dans un flacon en verre et placé à l'étuve à 105°C pendant 24 heures afin de décanter le glucose résiduel. Une dispersion à 5% massique dans l'eau montre une valeur de pH de 5,2 et le produit une couleur de 0,7 VCS. Le produit (266 g) est ensuite neutralisé à 90°C en réacteur sous agitation, en introduisant 0,1 g d'une solution aqueuse de Na₂CO₃ à 25 %.

Le produit est récupéré et référencé (Composition 5).

### Analyses :

- La valeur du pH d'une dispersion à 5% massique dans l'eau de la Composition 5 est de 6,5, et
- la mesure de la couleur de Composition 5 est de 0,7 VCS.

### Exemple 1.6 (tétradécanol-1 et Na₂CO₃ comme agent pré-neutralisant et Na₂CO₃ comme agent neutralisant) selon l'invention

### ETAPE 1 : Réaction de Glycosylation :

428,7 g d'alcool myristylique C-14 (ou tétradécanol-1) sont chargés dans un réacteur équipé d'une agitation mécanique et d'un montage de distillation sous vide. L'alcool est fondu à 80°C et mis sous agitation et sous barbotage d'azote. Le milieu est mis sous vide à 35 Torrs. 59,9 g de glucose anhydre sous forme de poudre sont ajoutés. Le milieu est inerté sous azote. Pour démarrer la réaction d'éthérification 1,0 g d'une solution aqueuse de H₃PO₂ à 50 % puis 0,7 g d'une solution aqueuse de H₂SO₄ à 98 % sont ajoutés et la température est augmentée et maintenue à 105°C. La réaction est poursuivie pendant 5h00.

### ETAPE 2 : Neutralisation du milieu réactionnel :

Le milieu est refroidi à 70°C à pression atmosphérique puis pré-neutralisé en introduisant 1,6 g d'une solution aqueuse de NaOH à 25 % sous agitation. Le produit est ensuite introduit dans un flacon en verre et placé à l'étuve à 80°C pendant 24 heures afin de décanter le glucose résiduel. Une dispersion à 5% massique dans l'eau montre une valeur de pH de 5,2 et le produit une couleur de 0,6 VCS. Le produit (337 g) est ensuite neutralisé à 70°C en réacteur sous agitation, en introduisant 0,7 g d'une solution aqueuse de Na₂CO₃ à 10 %. Le produit est récupéré et référencé (Composition 6).

### Analyses :

- La valeur du pH d'une dispersion à 5% massique dans l'eau de la Composition 6 est de 7,3, et
- la mesure de la couleur de Composition 6 est de 0,7 VCS.

### Exemple 1.7 (dodécanol-1 et Na₂CO₃ comme agent pré-neutralisant et Na₂CO₃ comme agent neutralisant) selon l'invention

### ETAPE 1 : Réaction de Glycosylation :

414,5 g d'alcool laurylique (C-12) (ou dodécanol-1) sont chargés dans un réacteur équipé d'une agitation mécanique et d'un montage de distillation sous vide. L'alcool est fondu à 85°C et mis sous agitation et sous barbotage d'azote. Le milieu est mis sous vide à 30 Torrs. 57,9 g de glucose anhydre sous forme de poudre sont ajoutés. Le milieu est inerté sous azote. Pour démarrer la réaction d'éthérification 0,4 g d'une solution aqueuse de H3PO2 à 50 % puis 0,7 g d'une solution aqueuse de H₂SO₄ à 98 % sont ajoutés et la température est augmentée et maintenue à 105°C. La réaction est poursuivie pendant 5h00.

### ETAPE 2 : Neutralisation du milieu réactionnel :

Le milieu est ensuite refroidi à 67°C à pression atmosphérique puis pré-neutralisé en introduisant 3,55 g d'une solution aqueuse de Na₂CO₃ à 10 %. Le produit est filtré sur filtre plaque (^{~} 100 µm) afin d'éliminer le glucose résiduel. Une dispersion à 5% massique dans l'eau montre une valeur de pH de 3,7 et le produit une couleur de 1,1 VCS. Le produit (197 g) est ensuite neutralisé en réacteur à 67°C en introduisant 2,97 g d'une solution aqueuse de Na₂CO₃ à 10 % sous agitation.

Le produit est récupéré et référencé (Composition 7).

### Analyses :

- La valeur du pH d'une dispersion à 5% massique dans l'eau de la Composition 7 est de 6,1, et
- la mesure de la couleur de Composition 6 est de 1,5 VCS.

### 2. Exemples comparatifs

### Exemple 2.1 : exemple comparatif (Coupe d'alcools 16/18 et NaOH comme agent pré-neutralisant et et NaOH comme agent neutralisant)

### ETAPE 1 : Réaction de Glycosylation :

778,1 g d'alcool cétéarylique (C-16/18) sont chargés dans un réacteur équipé d'une agitation mécanique et d'un montage de distillation sous vide. L'alcool est fondu à 85°C et mis sous agitation et sous barbotage d'azote. Le milieu est mis sous vide à des pressions inférieures à 50 Torrs. 102,2 g de glucose anhydre sous forme de poudre sont ajoutés. Le milieu est inerté sous azote. Pour démarrer la réaction d'éthérification 0,7 g d'une solution aqueuse de H₃PO₂ à 50 % puis 0,9 g d'une solution aqueuse de H₂SO₄ à 98 % sont ajoutés et la température est augmentée et maintenue à 105°C. La réaction est poursuivie pendant 5h45.

### ETAPE 2 : Neutralisation du milieu réactionnel :

Le milieu est ensuite refroidi à 80°C à pression atmosphérique puis pré-neutralisé en introduisant 1,86 g d'une solution aqueuse de NaOH à 25 %. Le produit est ensuite introduit dans un flacon en verre et placé à l'étuve à 80°C pendant 3 heures afin de décanter le glucose résiduel. Une dispersion à 5% massique dans l'eau montre une valeur de pH de 3,3 et le produit une couleur de 0,6 VCS.

Le produit est ensuite neutralisé à 80°C en introduisant 0,28 g d'une solution aqueuse de NaOH à 25 %. Le produit est récupéré et référencé (Composition 1').

### Analyses :

- La valeur du pH d'une dispersion à 5% massique dans l'eau de la Composition 1' est de 6,2, et
- la mesure de la couleur de Composition 1' est de 3,8 VCS.

### Exemple 2.2 : exemple comparatif (alcool C-12 et NaOH comme agent pré-neutralisant et et NaOH comme agent neutralisant)

### ETAPE 1 : Réaction de Glycosylation :

190,6 g d'alcool laurylique (C-12) (ou dodécanol-1) sont chargés dans un réacteur équipé d'une agitation mécanique et d'un montage de distillation sous vide. L'alcool est fondu à 85°C et mis sous agitation et sous barbotage d'azote. Le milieu est mis sous vide à 30 Torrs. 26,6 g de glucose anhydre sous forme de poudre sont ajoutés. Le milieu est inerté sous azote. Pour démarrer la réaction d'éthérification 0,2 g d'une solution aqueuse de H₃PO₂ à 50 % puis 0,3 g d'une solution aqueuse de H₂SO₄ à 98 % sont ajoutés et la température est augmentée et maintenue à 105°C. La réaction est poursuivie pendant 5h00.

### ETAPE 2 : Neutralisation du milieu réactionnel :

Le milieu est ensuite refroidi à 75°C à pression atmosphérique puis pré-neutralisé en introduisant 0,70 g d'une solution aqueuse de NaOH à 25 % sous agitation. Une dispersion à 5% massique dans l'eau montre une valeur de pH de 5,6 et le produit une couleur de 2,0 VCS. Le produit est filtré sur filtre K200 (^{~} 3-6 µm) afin d'éliminer le glucose résiduel. Le produit (78 g) est ensuite neutralisé en réacteur à 80°C en introduisant 0,08 g d'une solution aqueuse de NaOH à 25 % sous agitation.

Le produit est récupéré et référencé (Composition 2').

### Analyses :

- La valeur du pH d'une dispersion à 5% massique dans l'eau de la Composition 2' est de 6,5, et
- la mesure de la couleur de Composition 2' est de 4,5 VCS.

### 3. Observation et analyse des résultats

Le tableau 1 suivant rassemble des résultats du procédé de préparation de compositions (C) obtenues par la réaction entre le glucose et des alcools C-16/18 (mélange de 1-hexadécanol et de 1-octadécaniol). Les différents paramètres étudiés sont : *i)* la nature de l'agent de pré-neutralisation (Na₂CO₃ ou NaOH) et *ii)* la nature de l'agent de neutralisation final (Na₂CO₃, NaHCO₃ ou NaOH).

Les compositions référencées "Composition 1", "Composition 2" et "Composition 3" sont obtenues par la mise en oeuvre d'un procédé selon l'invention, et la composition référencée "Composition 1‴ est obtenue par la mise en oeuvre d'un procédé comparatif de l'état de la technique.

**Tableau 1**

| Référence | Composition 1' | Composition 1 | Composition 2 | Composition 3 |
|---|---|---|---|---|
| Agent Pré-neutralisant | NaOH | Na₂CO₃ | NaOH | NaOH |
| Agent Neutralisant | NaOH | Na₂CO₃ | Na₂CO₃ | NaHCO₃ |
| pH dispersion 5% dans l'eau | 6,2 | 6,8 | 7,2 | 6,3 |
| Couleur (vcs) | 3,8 | 0,5 | 0,9 | 0,4 |

Ces essais mettent en évidence que la présence d'une étape de pré-neutralisation par une solution aqueuse de NaOH et d'une étape de neutralisation par une solution aqueuse de NaOH, aboutit à une coloration trop élevée de la composition obtenue (jusqu'à 3,8 VCS pour la Composition 1'). En comparaison, lorsque l'étape de pré-neutralisation est réalisée avec une solution de NaOH (Composition 2) ou de Na₂CO₃ (Composition 1) et que l'étape de neutralisation est réalisée avec une solution aqueuse de Na₂CO₃ les couleurs des compositions obtenues sont très basses (< 1 vcs).

Pour la préparation de la Composition 3, le procédé selon l'invention a été mis en oeuvre en effectuant une étape de pré-neutralisation par une solution aqueuse de NaOH (pour atteindre un pH compris entre 3,5 et 5,5), puis une étape de neutralisation par une solution de NaHCO₃ après filtration (pour atteindre un pH compris entre 5,5 et 7,5).

Cet essai met en évidence qu'il est possible d'utiliser l'hydrogénocarbonate de sodium lors de l'étape de neutralisation du procédé selon l'invention pour garantir une faible couleur des Compositions recherchées (puisque la couleur finale obtenue est de 0,4 vcs pour la Composition 3).

Le tableau 2 rassemble des résultats du procédé de préparation de compositions (C) obtenues par la réaction entre le glucose et des alcools C-20/22 (mélange de 1-eicosanol et de 1-docosanol), entre le glucose et l'alcool C14 (ou 1-tétradécanol), entre le glucose et l'alcool C 12 (ou 1-dodécanol), en étudiant les mêmes paramètres que ceux impliqués dans l'étude ci-dessus.

**Tableau 2**

| Référence | Composition 4 | Composition 5 | Composition 6 | Composition 2' | Composition 7 |
|---|---|---|---|---|---|
| Chaîne AlkylPolyGlucoside | C-20/22 | | C-14 | C-12 | |
| Pré-neutralisant | NaOH | Na₂CO₃ | NaOH | NaOH | Na₂CO₃ |
| Neutralisant | Na₂CO₃ | Na₂CO₃ | Na₂CO₃ | NaOH | Na₂CO₃ |
| pH dispersion 5% dans l'eau | 6,8 | 6,5 | 7,3 | 6,5 | 6,1 |
| Couleur (vcs) | 0,5 | 0,7 | 0,7 | 4,5 | 1,5 |

Le procédé selon l'invention permet d'obtenir une composition (C), à faible coloration (inférieure ou égale à 1,5 VCS), préparée par la réaction du glucose et d'alcools gras comportant de 12, 14, 20 et 22 atomes de carbone.

En revanche, en mettant en œuvre un procédé de préparation de la Composition 2' (réaction entre le glucose et le 1-dodécanol) en utilisant la soude à la fois pour les étapes de pré-neutralisation et de neutralisation, les résultats analytiques obtenus montrent que la valeur de la couleur du mélange final est de 4,5 VCS, soit nettement supérieure à 1,5 VCS.

En conclusion, le procédé selon l'invention permet d'obtenir des compositions comprenant des alcools gras et des alkylpolyglycosides peu colorées (couleur inférieure ou égale à 1,5 VCS), à partir de la réaction d'au moins un sucre réducteur avec un alcool gras comportant de 12 à 22 atomes de carbone.

## Revendications

1. Procédé de préparation d'une composition (C) de couleur inférieure ou égale à 1,5 vcs comprenant pour 100% de sa masse :
i) une quantité supérieure ou égale à 40% massique et inférieure ou égale à 95% massique d'un alcool de formule (I) :
R-OH (I),
dans laquelle R représente un radical hydrocarboné, linéaire ou ramifié, saturé ou insaturé, pouvant comporter au moins un fonction hydroxy, et comportant de douze à vingt-deux atomes de carbone, ou d'un mélange d'alcools de formule (I) ;
ii) une quantité supérieure ou égale à 5% massique et inférieure ou égale à 60% massique d'une composition (C1) représentée par la formule (II) :
R-O-(G)x-H (II),
dans laquelle le reste G représente le reste d'un sucre réducteur, R représente un radical tel que défini dans la formule (I) et x, qui indique le degré moyen de polymérisation du reste G représente un nombre décimal supérieur à 1,05 et inférieur ou égal à 2,5, ou d'un mélange de compositions (C1) de formule (II);
étant entendu que la somme des proportions massiques des composés de formules (I) et (II) dans la composition (C) est égale à 100% massique,
ledit procédé comprenant successivement:
a) Une étape a) de glycosylation, consistant en une réaction entre au moins un alcool de formule (I) et au moins un sucre réducteur de formule (III) : H-O-(G)-H (III), en présence d'au moins un catalyseur acide (CA), à une température supérieure ou égale à 100°C et inférieure ou égale à 120°C, le au moins un catalyseur acide (CA) étant choisi parmi les éléments du groupe constitué par l'acide sulfurique, l'acide chlorhydrique, l'acide phosphorique, l'acide nitrique, l'acide hypophosphoreux, l'acide méthane-sulfonique, l'acide para-toluène sulfonique, l'acide trifluorométhane sulfonique et les résines échangeuses d'ions acides,
b) Une étape b) de pré-neutralisation du milieu réactionnel issu de l'étape a), la pré-neutralisation étant réalisée de manière à obtenir un milieu réactionnel dont une dispersion à 5% massique dudit milieu réactionnel dans l'eau présente une valeur de pH comprise entre 3,5 et 5,5,
c) Une étape c) d'élimination du sucre réducteur de formule (III), qui n'a pas réagi à l'étape a), du milieu réactionnel pré-neutralisé obtenu à l'étape b),
d) Une étape d) de neutralisation du milieu réactionnel issu de l'étape c) avec une solution aqueuse comprenant un agent basique (Ab) choisi parmi les éléments du groupe constitué par :
- les carbonates de formule (IVa) :
XnCO₃ (IVa),
dans laquelle X représente un atome de sodium ou de potassium et n est un nombre entier égal à 2, ou bien X représente un atome de calcium ou un atome de magnésium et n est un nombre entier égal à 1, ou
- les hydrogénocarbonates de formule (IVb) :
Y(HCO₃)m (IVb),
dans laquelle Y représente un atome de sodium ou de potassium et m est un nombre entier égal à 1, ou bien Y représente un atome de calcium ou un atome de magnésium et m est un nombre entier égal à 2,
la naturalisation étant réalisée de manière à obtenir un milieu réactionnel dont une dispersion à 5% massique dudit milieu réactionnel dans l'eau présente une valeur de pH comprise entre 5,5 et 7,5, et
e) Une étape e) de récupération d'au moins une composition (C) de couleur inférieure ou égale à 1,5 vcs.

2. Procédé selon la revendication 1, **caractérisé en ce que** ladite composition (C1) consiste en un mélange de composés représentés par les formules (II1), (112), (II3), (II4) et (115):
R-O-(G)1-H (II1),
R-O-(G)2-H (II2),
R-O-(G)3-H (II3),
R-O-(G)4-H (II4),
R-O-(G)5-H (II5),
dans les proportions molaires respectives a1, a2, a3, a4 et a5, telles que:
▪ la somme: a1+ a2 + a3 + a4 + a5 est égale à 1, et
▪ la somme a1 + 2a2 + 3a3 + 4a4 + 5a5 est égale à x.

3. Procédé selon l'une des revendications 1 ou 2, **caractérisé en ce que** la composition (C) comprend une quantité inférieure ou égale à 2% massique, plus particulièrement inférieure ou égale à 1% massique du sucre réducteur de formule (III) :
H-O-(G)-H (III) ;
étant entendu que la somme des proportions massiques des composés de formules (I), (II) et (III) dans la composition (C) est égale à 100% massique.

4. Procédé selon l'une des revendications 1 à 3, **caractérisé en ce que** l'agent basique (Ab) compris dans la solution aqueuse utilisée dans l'étape d) de neutralisation est choisi parmi le carbonate de sodium (Na₂CO₃) ou l'hydrogénocarbonate de sodium (NaHCO₃).

5. Procédé selon l'une des revendications 1 à 4, **caractérisé en ce que** l'étape b) de pré-neutralisation est réalisée avec au moins un des composés suivants : hydroxyde de sodium (NaOH), hydroxyde de potassium (KOH), hydroxyde d'ammonium (NH₄OH), monoéthanolamine, diéthanolamine, triéthanolamine et triéthylamine.

6. Procédé selon l'une des revendications 1 à 5, **caractérisée en ce que** l'étape b) de pré-neutralisation est réalisée avec un des composés suivants : carbonate de sodium (Na₂CO₃), hydrogénocarbonate de sodium (NaHCO₃), et carbonate de calcium (CaCO₃).

7. Procédé selon l'une des revendications 1 à 6, **caractérisé en ce que** le sucre réducteur de formule (III) choisi pour la glycolysation de l'étape a) est choisi parmi les éléments du groupe constitué par le glucose, le xylose, l'arabinose, le rhamnose.

8. Procédé selon l'une des revendications 1 à 7, **caractérisé en ce que** l'étape c) d'élimination du sucre réducteur de formule (III) est réalisée par filtration, centrifugation ou décantation.

9. Procédé selon l'une des revendications 1 à 8, **caractérisé en ce qu'**à l'étape d) la solution aqueuse d'agent basique (Ab) comprend entre 10 % et 25% massique dudit agent basique (Ab).

10. Procédé selon l'une des revendications 1 à 9, **caractérisé en ce que** l'étape a) comprend les sous-étapes successives suivantes :
i) Introduction d'un alcool de formule (I) ou d'un mélange d'alcools de formule (I), dans un réacteur (Ré) équipé d'une agitation mécanique et d'un dispositif de mise sous vide ;
ii) Chauffage de l'alcool de formule (I) à une température comprise entre 80°C et 90°C sous agitation mécanique;
iii) Chargement du sucre réducteur de formule (III) dans le réacteur (Ré) ;
iv) Introduction du catalyseur acide (CA) dans le réacteur (Ré),
v) Chauffage sous vide partiel du milieu réactionnel issu de la sous-étape iv) et présent dans le réacteur (Ré) à une température comprise entre 100°Cet 110°C pendant la durée de la réaction, et
vi) Refroidissement du milieu réactionnel issu de la sous-étape v) à une température comprise entre70°C et 80°C.

11. Procédé selon la revendication 10, **caractérisé en ce que** le radical R est choisi parmi les radicaux suivants : lauryle (ou n-dodécyle), myristyle (ou n-tétradécyle ), n-pentadécyle, cétyle (ou n-hexadécyle), n-heptadécyle, stéaryle (ou n-octadécyle), palmitoléyle (ou 9-hexadécényle), oléyle (ou 9-octadécényle), linoléyle (9,12-octadecadiényle), linolényle (ou 6,9,12-octadécatriényle) nonadécyle, arachidyle (ou n-eicosyle), béhényle (ou n-docosyle), érucyle (13-docosényle), ou 12-hydroxystéaryle.

12. Procédé selon la revendication 10, **caractérisé en ce que** le radical R est choisi parmi les radicaux suivants : 2-hexyl octyle, 2-hexyl décyle, 2-hexyl dodécyle, 2-octyl décyle, 2-octyl dodécyle, 2-décyl tétradécyle, isostéaryle (ou 16-méthyl heptadécyle) ou isomyristyle (ou 13-méthyl tridécyle).

13. Procédé selon l'une des revendications 10 à 12, **caractérisé en ce que** pendant les sous-étapes ii) à iv) le réacteur (Ré) est inerté sous azote.

14. Procédé selon l'une des revendications 10 à 13, **caractérisé en ce qu'**il comprend entre les sous-étapes ii) et iii) une étape de mise sous vide, de préférence à une pression inférieure à ou égale à 50 millibars.

15. Procédé selon l'une des revendications 10 à 14, **caractérisé en ce que** la sous-étape vi) est réalisée à pression atmosphérique.

## Patentansprüche

1. Verfahren zur Herstellung einer Zusammensetzung (C) mit einer Farbe von höchstens 1,5 VCS, umfassend, bezogen auf 100 Gew.-% ihrer Masse:
i) eine Menge von mindestens 40 Gew.-% und höchstens 95 Gew.-% eines Alkohols der Formel (I):
R-OH (I),
wobei R einen Kohlenwasserstoffrest, linear oder verzweigt, gesättigt oder ungesättigt, der mindestens eine Hydroxyfunktion aufweisen kann und zwölf bis zweiundzwanzig Kohlenstoffatome umfasst, oder eine Mischung von Alkoholen der Formel (I) darstellt;
ii) eine Menge von mindestens 5 Gew.-% und höchstens 60 Gew.-% einer Zusammensetzung (C1), dargestellt durch die Formel (II):
R-O-(G)x-H (II),
wobei der Rest G den Rest eines reduzierenden Zuckers darstellt, R einen Rest darstellt, wie er in Formel (I) definiert ist, und x, welches den mittleren Polymerisationsgrad des Restes G angibt, eine Dezimalzahl größer oder gleich 1,05 und kleiner oder gleich 2,5 darstellt, oder eine Mischung von Zusammensetzungen (C1) der Formel (II);
wobei gilt, dass die Summe der Massenanteile der Verbindungen der Formeln (I) und (II) in der Zusammensetzung (C) 100 Gew.-% beträgt,
wobei das Verfahren nacheinander umfasst:
a) Einen Glykosylierungsschritt a), bestehend aus einer Reaktion zwischen mindestens einem Alkohol der Formel (I) und mindestens einem reduzierenden Zucker der Formel (III): H-O-(G)-H (III), in Gegenwart mindestens eines sauren Katalysators (CA), bei einer Temperatur von mindestens 100°C und höchstens 120°C, wobei der mindestens eine saure Katalysator (CA) ausgewählt ist aus den Elementen der Gruppe bestehend aus Schwefelsäure, Salzsäure, Phosphorsäure, Salpetersäure, hypophosphoriger Säure, Methansulfonsäure, p-Toluolsulfonsäure, Trifluormethansulfonsäure und Ionenaustauscherharzen,
b) Einen Vorneutralisierungsschritt b) des aus Schritt a) stammenden Reaktionsmediums, wobei die Vorneutralisierung derart durchgeführt wird, dass ein Reaktionsmedium erhalten wird, dessen 5 Gew.-% Dispersion in Wasser einen pH-Wert zwischen 3,5 und 5,5 aufweist,
c) Einen Schritt c) der Eliminierung des in Schritt a) nicht umgesetzten reduzierenden Zuckers der Formel (III) aus dem in Schritt b) erhaltenen vorneutralisierten Reaktionsmedium,
d) Einen Neutralisierungsschritt d) des aus Schritt c) stammenden Reaktionsmediums mit einer wässrigen Lösung, umfassend ein basisches Mittel (Ab), ausgewählt aus den Elementen der Gruppe bestehend aus:
- den Carbonaten der Formel (IVa):
XnCO₃ (IVa),
wobei X ein Natrium- oder Kaliumatom darstellt und n eine ganze Zahl gleich 2 ist, oder X ein Calciumatom oder ein Magnesiumatom darstellt und n eine ganze Zahl gleich 1 ist, oder
- den Hydrogencarbonaten der Formel (IVb):
Y(HCO₃)m (IVb),
wobei Y ein Natrium- oder Kaliumatom darstellt und m eine ganze Zahl gleich 1 ist, oder Y ein Calciumatom oder ein Magnesiumatom darstellt und m eine ganze Zahl gleich 2 ist,
wobei die Neutralisierung derart durchgeführt wird, dass ein Reaktionsmedium erhalten wird, dessen 5 Gew.-% Dispersion in Wasser einen pH-Wert zwischen 5,5 und 7,5 aufweist, und
e) Einen Schritt e) der Gewinnung mindestens einer Zusammensetzung (C) mit einer Farbe von höchstens 1,5 VCS.

2. Verfahren nach Anspruch 1, **dadurch gekennzeichnet, dass** die Zusammensetzung (C1) aus einer Mischung von Verbindungen besteht, die durch die Formeln (II1), (II2), (II3), (II4) und (II5) dargestellt sind:
R-O-(G)1-H (II1),
R-O-(G)2-H (II2),
R-O-(G)3-H (II3),
**R-O-(G)4-H** (II4),
R-O-(G)5-H (II5),
in den jeweiligen molaren Anteilen a1, a2, a3, a4 und a5, so dass:
- die Summe: a1+ a2 + a3 + a4 + a5 gleich 1 ist, und
- die Summe a1 + 2a2 + 3a3 + 4a4 + 5a5 gleich x ist.

3. Verfahren nach einem der Ansprüche 1 oder 2, **dadurch gekennzeichnet, dass** die Zusammensetzung (C) eine Menge von höchstens 2 Gew.-%, insbesondere höchstens 1 Gew.-% des reduzierenden Zuckers der Formel (III):
H-O-(G)-H (III)
umfasst;
wobei gilt, dass die Summe der Massenanteile der Verbindungen der Formeln (I), (II) und (III) in der Zusammensetzung (C) 100 Gew.-% beträgt.

4. Verfahren nach einem der Ansprüche 1 bis 3, **dadurch gekennzeichnet, dass** das in der in Schritt d) verwendeten wässrigen Lösung enthaltene basische Mittel (Ab) ausgewählt ist aus Natriumcarbonat (Na₂CO₃) oder Natriumhydrogencarbonat (NaHCO₃).

5. Verfahren nach einem der Ansprüche 1 bis 4, **dadurch gekennzeichnet, dass** der Vorneutralisierungsschritt b) mit mindestens einer der folgenden Verbindungen durchgeführt wird: Natriumhydroxid (NaOH), Kaliumhydroxid (KOH), Ammoniumhydroxid (NH₄OH), Monoethanolamin, Diethanolamin, Triethanolamin und Triethylamin.

6. Verfahren nach einem der Ansprüche 1 bis 5, **dadurch gekennzeichnet, dass** der Vorneutralisierungsschritt b) mit einer der folgenden Verbindungen durchgeführt wird: Natriumcarbonat (Na₂CO₃), Natriumhydrogencarbonat (NaHCO₃) und Calciumcarbonat (CaCO₃).

7. Verfahren nach einem der Ansprüche 1 bis 6, **dadurch gekennzeichnet, dass** der für die Glykosylierung von Schritt a) ausgewählte reduzierende Zucker der Formel (III) ausgewählt ist aus den Elementen der Gruppe bestehend aus Glucose, Xylose, Arabinose, Rhamnose.

8. Verfahren nach einem der Ansprüche 1 bis 7, **dadurch gekennzeichnet, dass** Schritt c) der Eliminierung des reduzierenden Zuckers der Formel (III) durch Filtration, Zentrifugation oder Dekantierung durchgeführt wird.

9. Verfahren nach einem der Ansprüche 1 bis 8, **dadurch gekennzeichnet, dass** in Schritt d) die wässrige Lösung des basischen Mittels (Ab) zwischen 10 Gew.-% und 25 Gew.-% des besagten basischen Mittels (Ab) umfasst.

10. Verfahren nach einem der Ansprüche 1 bis 9, **dadurch gekennzeichnet, dass** Schritt a) die folgenden aufeinanderfolgenden Unterschritte umfasst:
i) Einbringen eines Alkohols der Formel (I) oder einer Mischung von Alkoholen der Formel (I) in einen Reaktor (Ré), der mit einem mechanischen Rührwerk und einer Vakuumdestillationsvorrichtung ausgestattet ist;
ii) Erhitzen des Alkohols der Formel (I) auf eine Temperatur zwischen 80°C und 90°C unter mechanischem Rühren;
iii) Beschickung des reduzierenden Zuckers der Formel (III) in den Reaktor (Ré);
iv) Einbringen des sauren Katalysators (CA) in den Reaktor (Ré),
v) Erhitzen unter Teilvakuum des aus Unterschritt iv) stammenden und im Reaktor (Ré) vorhandenen Reaktionsmediums auf eine Temperatur zwischen 100°C und 110°C während der Dauer der Reaktion, und
vi) Abkühlen des aus Unterschritt v) stammenden Reaktionsmediums auf eine Temperatur zwischen 70°C und 80°C.

11. Verfahren nach Anspruch 10, **dadurch gekennzeichnet, dass** der Rest R ausgewählt ist aus den folgenden Resten: Lauryl (oder n-Dodecyl), Myristyl (oder n-Tetradecyl), n-Pentadecyl, Cetyl (oder n-Hexadecyl), n-Heptadecyl, Stearyl (oder n-Octadecyl), Palmitoleyl (oder 9-Hexadecenyl), Oleyl (oder 9-Octadecenyl), Linoleyl (9,12-Octadecadienyl), Linolenyl (oder 6,9,12-Octadecatrienyl) Nonadecyl, Arachidyl (oder n-Eicosyl), Behenyl (oder n-Docosyl), Erucyl (13-Docosenyl) oder 12-Hydroxystearyl.

12. Verfahren nach Anspruch 10, **dadurch gekennzeichnet, dass** der Rest R ausgewählt ist aus den folgenden Resten: 2-Hexyloctyl, 2-Hexyldecyl, 2-Hexyldodecyl, 2-Octyldecyl, 2-Octyldodecyl, 2-Decyltetradecyl, Isostearyl (oder 16-Methylheptadecyl) oder Isomyristyl (oder 13-Methyltridecyl).

13. Verfahren nach einem der Ansprüche 10 bis 12, **dadurch gekennzeichnet, dass** während der Unterschritte ii) bis iv) der Reaktor (Ré) unter Stickstoff inertisiert wird.

14. Verfahren nach einem der Ansprüche 10 bis 13, **dadurch gekennzeichnet, dass** es zwischen den Unterschritten ii) und iii) einen Schritt des Anlegens eines Vakuums umfasst, vorzugsweise bei einem Druck von höchstens 50 Millibar.

15. Verfahren nach einem der Ansprüche 10 bis 14, **dadurch gekennzeichnet, dass** der Unterschritt vi) bei atmosphärischem Druck durchgeführt wird.

## Claims

1. A Process for the preparation of a composition (C) having a colour lower than or equal to 1.5 vcs, comprising, for 100% of its mass:
i) a quantity greater than or equal to 40% by mass and less than or equal to 95% by mass of an alcohol of formula (I):
R-OH (I),
in which R represents a hydrocarbon radical, linear or branched, saturated or unsaturated, possibly comprising at least one hydroxyl function, and comprising twelve to twenty-two carbon atoms, or a mixture of alcohols of formula (I);
ii) a quantity greater than or equal to 5% by mass and less than or equal to 60% by mass of a composition (C1) represented by formula (II):
R-O-(G)x-H (II),
in which the residue G represents the residue of a reducing sugar, R represents a radical as defined in formula (I) and x, which indicates the average degree of polymerization of the residue G, represents a decimal number greater than or equal to 1.05 and less than or equal to 2.5, or a mixture of compositions (C1) of formula (II);
it being understood that the sum of the mass proportions of the compounds of formulae (I) and (II) in the composition (C) is equal to 100% by mass,
said process successively comprising:
a) A glycosylation step a), consisting of a reaction between at least one alcohol of formula (I) and at least one reducing sugar of formula (III): H-O-(G)-H (III), in the presence of at least one acid catalyst (CA), at a temperature greater than or equal to 100°C and less than or equal to 120°C, the at least one acid catalyst (CA) being chosen from the elements of the group consisting of sulfuric acid, hydrochloric acid, phosphoric acid, nitric acid, hypophosphorous acid, methane-sulfonic acid, para-toluene sulfonic acid, trifluoromethane sulfonic acid and ion exchange resins,
b) A pre-neutralization step b) of the reaction medium resulting from step a), the pre-neutralization being carried out so as to obtain a reaction medium, a 5% by mass dispersion of which in water exhibits a pH value comprised between 3.5 and 5.5,
c) A step c) of eliminating the reducing sugar of formula (III), which has not reacted in step a), from the pre-neutralized reaction medium obtained in step b),
d) A neutralization step d) of the reaction medium resulting from step c) with an aqueous solution comprising a basic agent (Ab) chosen from the elements of the group consisting of:
- carbonates of formula (IVa):
XnCO₃ (IVa),
wherein X represents a sodium or potassium atom and n is an integer equal to 2, or X represents a calcium atom or a magnesium atom and n is an integer equal to 1, or
- hydrogencarbonates of formula (IVb):
Y(HCO₃)m (IVb),
wherein Y represents a sodium or potassium atom and m is an integer equal to 1, or Y represents a calcium atom or a magnesium atom and m is an integer equal to 2,
the neutralization being carried out so as to obtain a reaction medium, a 5% by mass dispersion of which in water exhibits a pH value comprised between 5.5 and 7.5, and
e) A step e) of recovering at least one composition (C) having a colour lower than or equal to 1.5 vcs.

2. The Process according to claim 1, **characterized in that** said composition (C1) consists of a mixture of compounds represented by the formulae (III), (II2), (113), (II4) and (II5):
R-O-(G)1-H (II1),
R-O-(G)2-H (II2),
R-O-(G)3-H (II3),
R-O-(G)4-H (II4),
R-O-(G)5-H (II5),
in the respective molar proportions a1, a2, a3, a4 and a5, such that:
- the sum: a1+ a2 + a3 + a4 + a5 is equal to 1, and
- the sum a1 + 2a2 + 3a3 + 4a4 + 5a5 is equal to x.

3. The Process according to any one of claims 1 or 2, **characterized in that** the composition (C) comprises a quantity less than or equal to 2% by mass, more particularly less than or equal to 1% by mass of the reducing sugar of formula (III):
H-O-(G)-H (III);
it being understood that the sum of the mass proportions of the compounds of formulae (I), (II) and (III) in the composition (C) is equal to 100% by mass.

4. The Process according to any one of claims 1 to 3, **characterized in that** the basic agent (Ab) comprised in the aqueous solution used in neutralization step d) is chosen from sodium carbonate (Na₂CO₃) or sodium hydrogencarbonate (NaHCO₃).

5. The Process according to any one of claims 1 to 4, **characterized in that** the pre-neutralization step b) is carried out with at least one of the following compounds: sodium hydroxide (NaOH), potassium hydroxide (KOH), ammonium hydroxide (NH₄OH), monoethanolamine, diethanolamine, triethanolamine and triethylamine.

6. The Process according to any one of claims 1 to 5, **characterized in that** the pre-neutralization step b) is carried out with one of the following compounds: sodium carbonate (Na₂CO₃), sodium hydrogencarbonate (NaHCO₃), and calcium carbonate (CaCO₃).

7. The Process according to any one of claims 1 to 6, **characterized in that** the reducing sugar of formula (III) chosen for the glycosylation of step a) is chosen from the elements of the group consisting of glucose, xylose, arabinose, rhamnose.

8. The Process according to any one of claims 1 to 7, **characterized in that** step c) of eliminating the reducing sugar of formula (III) is carried out by filtration, centrifugation or decantation.

9. The Process according to any one of claims 1 to 8, **characterized in that** in step d) the aqueous solution of basic agent (Ab) comprises between 10% and 25% by mass of said basic agent (Ab).

10. The Process according to any one of claims 1 to 9, **characterized in that** step a) comprises the following successive sub-steps:
i) Introduction of an alcohol of formula (I) or a mixture of alcohols of formula (I), into a reactor (Ré) equipped with mechanical stirring and a vacuum distillation device;
ii) Heating the alcohol of formula (I) to a temperature comprised between 80°C and 90°C under mechanical stirring;
iii) Charging the reducing sugar of formula (III) into the reactor (Ré);
iv) Introduction of the acid catalyst (CA) into the reactor (Ré),
v) Heating under partial vacuum of the reaction medium resulting from sub-step iv) and present in the reactor (Ré) at a temperature comprised between 100°C and 110°C during the duration of the reaction, and
vi) Cooling the reaction medium resulting from sub-step v) to a temperature comprised between 70°C and 80°C.

11. The Process according to claim 10, **characterized in that** the radical R is chosen from the following radicals: lauryl (or n-dodecyl), myristyl (or n-tetradecyl), n-pentadecyl, cetyl (or n-hexadecyl), n-heptadecyl, stearyl (or n-octadecyl), palmitoleyl (or 9-hexadecenyl), oleyl (or 9-octadecenyl), linoleyl (9,12-octadecadienyl), linolenyl (or 6,9,12-octadecatrienyl) nonadecyl, arachidyl (or n-eicosyl), behenyl (or n-docosyl), erucyl (13-docosenyl), or 12-hydroxystearyl.

12. The Process according to claim 10, **characterized in that** the radical R is chosen from the following radicals: 2-hexyl octyl, 2-hexyl decyl, 2-hexyl dodecyl, 2-octyl decyl, 2-octyl dodecyl, 2-decyl tetradecyl, isostearyl (or 16-methyl heptadecyl) or isomyristyl (or 13-methyl tridecyl).

13. The Process according to any one of claims 10 to 12, **characterized in that** during sub-steps ii) to iv) the reactor (Ré) is purged under nitrogen.

14. The Process according to any one of claims 10 to 13, **characterized in that** it comprises between sub-steps ii) and iii) a vacuum application step, preferably at a pressure less than or equal to 50 millibars.

15. The Process according to any one of claims 10 to 14, **characterized in that** sub-step vi) is carried out at atmospheric pressure.
